# EUROPÄISCHE PATENTANMELDUNG

(11) **EP 1 992 604 A2**
(43) Veröffentlichungstag der Anmeldung: **19.11.2008**
(21) Anmeldenummer: 08008078.1
(22) Anmeldetag: 26.04.2008
(51) Int. Cl.: C07C 37/84, C07C 37/20, C07C 39/16

(54) **Verfahren zur Herstellung von Bisphenol A mit verlängerter Standzeit in der Kristallisation**

(30) Priorität: 10.05.2007 DE 102007021935
(71) Anmelder: Bayer MaterialScience AG, 51368 Leverkusen (DE)
(72) Erfinder: Blaschke, Ulrich, Dr., 47829 Krefeld (DE); Audenaert, Raymond, Dr., 9220 Hamme (BE); Geers, Jeroen, 4631 GN Hoogerheide (NL); de Kock, Philip, 2950 Kapellen (BE); Salembier, Paul, 9140 Tielrode (BE); Petein, Dirk, 9150 Kruibeke (BE)

(57) **Zusammenfassung**

Die Erfindung beschreibt ein Verfahren zur Herstellung von Bisphenol mit einem Gehalt von weniger als 15 Gew.-% Phenol, bei dem eine Suspensionskristallisation eines Produktgemisches enthaltend Bisphenol A, Nebenkomponenten und Phenol in einem Kristaller durchgeführt wird, wobei das Produktgemisch über einen Wärmetauscher gepumpt wird. Durch Ablagerung (Fouling, dass heisst Kristallisation und Ablagerung auf der Oberfläche der Wärmetauscher) in dem Wärmetauscher steigt die Druckdifferenz von 0,5 bis auf 3 bar über den Wärmetauscher. Die dadurch bedingte verringerte Strömungsgeschwindigkeit, die zu einer verstärkten Fouling im Kristaller führen würde, wird durch eine kontinuierliche Erhöhung der Drehzahl der Pumpe ausgeglichen, wobei die Regelung der Pumpendrehzahl dadurch erfolgt, dass die Stromaufnahme der Pumpe so gehalten wird, dass sie um maximal ± 5% schwankt.

## Beschreibung

Die Erfindung beschreibt ein Verfahren zur Herstellung von Bisphenol mit einem Gehalt von weniger als 15 Gew.-% Phenol, bei dem eine Suspensionskristallisation eines Produktgemisches enthaltend Bisphenol A, Nebenkomponenten und Phenol in einem Kristaller durchgeführt wird, wobei das Produktgemisch über einen Wärmetauscher gepumpt wird. Durch Ablagerung (Fouling, dass heisst Kristallisation und Ablagerung auf der Oberfläche der Wärmetauscher) in dem Wärmetauscher steigt die Druckdifferenz von 1 bis auf 3 bar über den Wärmetauscher. Die dadurch bedingte verringerte Strömungsgeschwindigkeit, die zu einer verstärkten Fouling im Kristaller führen würde, wird durch eine kontinuierliche Erhöhung der Drehzahl der Pumpe ausgeglichen, wobei die Regelung der Pumpendrehzahl dadurch erfolgt, dass die Stromaufnahme so gehalten wird, dass sie um maximal ± 5% schwankt.

Bisphenole als Kondensationsprodukte von Phenolen und Carbonylverbindungen sind Ausgangsstoffe oder Zwischenprodukte zur Herstellung einer Vielzahl kommerzieller Produkte. Von besonderer technischer Bedeutung ist das Kondensationsprodukt aus der Reaktion zwischen Phenol und Aceton, 2,2-Bis(4-hydroxyphenyl)propan (Bisphenol A, BPA). BPA dient als Ausgangsstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxydharzen und Polycarbonaten.

Technisch relevante Herstellmethoden für BPA sind bekannt und beruhen auf der säurekatalysierten Umsetzung von Phenol mit Aceton und sind beispielsweise in US 2 775 620 und in EP-A 0 342 758 beschrieben.

Bei der säurekatalysierten Umsetzung von Phenol mit Aceton wird bevorzugt ein Phenol-Aceton-Verhältnis von größer 5 : 1 in der Reaktion eingestellt. Die Reaktion erfolgt dabei üblicherweise im Dauerbetrieb und im Allgemeinen bei Temperaturen von 45 bis 110°C. Als saure Katalysatoren können homogene wie auch heterogene Brönstedt- oder Lewissäuren genutzt werden, so beispielsweise starke Mineralsäuren wie Salz- oder Schwefelsäure. Bevorzugt kommen gelförmige oder makroporöse sulfonierte vernetzte Polystyrolharze (saure Ionentauscher) zum Einsatz, die als Vernetzer Divinylbenzol enthalten. Neben dem Katalysator kommt in der Regel ein Thiol als Cokatalysator zum Einsatz.

Bei der Umsetzung von Phenol mit Aceton in Gegenwart saurer Katalysatoren entsteht ein Produktgemisch, das neben nicht umgesetztem Phenol und gegebenenfalls Aceton in erster Linie BPA und Wasser enthält. Daneben treten typische Nebenprodukte der Kondensationsreaktion auf, so beispielsweise 2-(4-Hydroxyphenyl)-2-(2-hydroxyphenyl)propan (o,p-BPA), substituierte Indane, Hydroxyphenylindanole, Hydroxyphenylchromane, Spirobisindane, substituierte Indenole, substituierte Xanthene und höher kondensierte Verbindungen mit drei oder mehr Phenylringen im Molekülgerüst. Außerdem können sich durch Eigenkondensation des Acetons und Reaktion mit Verunreinigungen in den Rohstoffen weitere Nebenkomponenten wie Anisol, Mesityloxid, Mesitylen und Diacetonalkohol bilden.

Die genannten Nebenprodukte wie Wasser, aber auch die nicht umgesetzten Einsatzstoffe wie Phenol und Aceton beeinträchtigen die Eignung von BPA zur Herstellung von Polymeren und müssen durch geeignete Verfahren abgetrennt werden. Insbesondere zur Herstellung von Polycarbonat werden hohe Reinheitsanforderungen an den Rohstoff BPA gestellt.

Eine Aufarbeitungs- und Reinigungsmethode von BPA erfolgt durch Abtrennung von BPA aus dem Produktgemisch in Form eines etwa äquimolaren kristallinen Addukts mit Phenol durch Abkühlen der Reaktionsmischung unter Auskristallisieren des BPA-Phenol-Addukts in einer Suspensionskristallisation. Die BPA-Phenol-Adduktkristalle werden anschließend durch eine geeignete Apparatur zur Fest-Flüssigtrennung wie Drehfilter oder Zentrifugen von der Flüssigphase abgetrennt und der weiteren Reinigung zugeführt.

Bei der Suspensionskristallisation wird dem Bisphenol A und Phenol enthaltenden Produktgemisch in einem oder mehreren Kühlern kontinuierlich oder halbkontinuierlich Wärme entzogen und so eine Übersättigung hergestellt, dass es in der Folge zum Auskristallisieren der BPA-Phenol-Adduktkristalle kommt. Die zum Abbau der Übersättigung und damit dem Auskristallisieren notwendige Verweilzeit wird zusätzlich zu den Kühlern in einem Kristaller bereitgestellt. Die Suspension des Kristallers wird in der Regel durch Pumpen durch den oder die Kühler umgewälzt. Aus der Suspension wird dabei kontinuierlich oder teilkontinuierlich ein Strom zur weiteren Aufarbeitung ausgespeist, ebenso wird frisches Produktgemisch aus der Reaktion nachgeführt, wobei dem Produktgemisch vorher durch Destillation Wasser, Aceton und andere leichtflüchtige Komponenten wie beispielsweise Cokatalysator ganz oder teilweise entzogen werden können.

Verfahren zur Herstellung von Bisphenolen mit einer solchen Suspensionskristallisation sind in DE-A 100 15 014 und in DE-A 195 10 063 beschrieben.

Hierin ist auch beschrieben, dass es bei einer solchen Suspensionskristallisation mittels Umwälzkühler zur Abscheidung von Belägen (so genanntes Fouling) bestehend aus beispielsweise Bisphenol oder BPA-Phenol-Adduktkristallen auf der Innenfläche der suspensionsführenden Rohre kommt. Die an der Innenfläche der suspensionsführenden Rohre anhaftenden Produkte müssen in regelmäßigen Abständen durch Aufheizen auf Temperaturen über 80°C entfernt werden (so genanntes Aufschmelzen), da Sie den Wärmeübergang zwischen Kühler und Suspension und den Durchfluss durch die Rohre verschlechtern.

US 5,856,589 beschreibt die Entfernung solcher Beläge mittels Phenol mit einem Wassergehalt von 5 bis 40 Gew.-%.

US 6,203,612 beschreibt ein weiteres, kompliziertes Verfahren zum Entfernen der Beläge, bei dem ein Teil der im Kristaller bzw. Kühler enthaltenen Suspension zunächst durch Phenol ersetzt und die verdünnte Suspension erhitzt wird, bis die Kristalle gelöste sind. Anschließend wird schnell abgekühlt und Impfkristalle werden zugesetzt.

WO-A 00/47542 beschreibt das Betreiben einer Suspensionskristallisation, bei der ein Kristaller zwei Kühler enthält, wobei zum Entfernen der Beläge ein Kühler außer Betrieb genommen und von den Belägen durch Erhöhen der Temperatur befreit wird, während der andere Kühler in Betrieb bleibt und dessen Temperatur dabei um mindestens 1°C abgesenkt wird.

Diese Anmeldungen beschäftigen sich allesamt mit dem Entfernen der Beläge. Während dieser Zeit muss die Produktion des Bisphenols reduziert oder ganz eingestellt werden.

Aufgabe dieser Erfindung war es daher, ein Verfahren bereit zustellen, das es ermöglicht, die Zeitabstände zwischen den Maßnahmen zur Entfernung der Beläge zu erhöhen.

Diese Aufgabe wird gelöst durch ein Verfahren zur Herstellung von Bisphenol A umfassend folgende Schritte:
a) Umsetzung von Phenol und Aceton in Gegenwart eines sulfonsauren Ionenaustauschers und eines schwefelhaltigen Cokatalysators zu einem Bisphenol A enthaltenden Produktgemisch,
b) kontinuierliche Suspensionskristallisation eines Bisphenol A-Phenol-Addukts aus dem in Schritt (a) erhaltenen Produktgemisch in einem oder mehreren Kristallern mit jeweils mindestens einem Wärmetauscher zur Kühlung und mindestens einer Pumpe, die die in den Kristallern enthaltene Suspension durch den oder die Wärmetauscher pumpt,
c) Abtrennung der so gewonnenen BPA-Phenol-Adduktkristalle durch Fest-Flüssig-Trennung,
d) Entfernung von Phenol mittels Destillation und/oder Desorption aus dem gemäß Schritt (c) erhaltenen BPA-Phenol-Addukt
dadurch gekennzeichnet, dass die Stromaufnahme der Pumpe(n), mit denen das Produktgemisch in Schritt b) durch die Wärmetauscher gepumpt wird, so gehalten wird, dass die Stromaufnahme um maximal ± 5% schwankt und damit mit steigendem Druckverlust über die Wärmetauscher die Drehzahl der Pumpen erhöht wird.

Vorteilhaft bei dem erfindungsgemäßen Verfahren wird das Produkt aus Schritt c) mit Phenol gewaschen und/oder umkristallisiert.

Vorteilhaft ist eine Drehzahlerhöhung der Pumpen über das gesamte erfindungsgemäße Verfahren von Inbetriebnahme der Wärmetauscher bis zu einem Entfernen der Beläge im Wärmetauscher von 10 - 50 %. So liegt beispielsweise die Drehzahl der Pumpe nach einem Abschmelzvorgang (= Inbetriebnahme) bei 450 U / min, nach Erreichen von 600 U / min erfolgt ein Abschmelzen der Wärmetauscher.

Durch die konstante Stromaufnahme soll der umgewälzte Volumenstrom durch den oder die eingesetzten Wärmetauschers um maximal 15% schwanken.

Vorteilhaft schwankt bei dem erfindungsgemäßen Verfahren die Stromaufnahme der Pumpe für den Wärmetauscher um maximal 5%.

Alternativ sind Verfahren, bei denen die Steuerung der Pumpendrehzal über den Volumenstrom oder über die Druckdifferenz über den Wärmeaustauscher erfolgt, vorteilhaft und einfacher ist jedoch die Steuerung über die Stromaufnahme der Pumpe.

Die Erfindung betrifft daher auch ein Verfahren zur Herstellung von Bisphenol A, bei dem aus Bisphenol A und Phenol enthaltenden Produktgemischen BPA-Phenol-Adduktkristalle durch eine kontinuierliche oder halbkontinuierliche Suspensionskristallisation derart auskristallisiert werden, dass dem Produktgemisch durch Umpumpen dieses Produktgemisches durch einen oder mehrere Wärmetauscher Wärme entzogen wird, und dem Produktgemisch in einem Kristaller Zeit gegeben wird, um die so entstandene Übersättigung abzubauen, wobei die Druckdifferenz Δp und/oder der Volumenstrom im Wärmetauscher des oder der Kühler gemessen werden und die Drehzahl der Pumpe zur Umwälzung des Produktgemisches erhöht wird, sobald ein Anstieg der Druckdifferenz oder ein Abfall des Volumenstromes gemessen wird.

Alle dem Fachmann bekannten Chemie-Normpumpen sind für das erfindungsgemäße Verfahren einsetzbar. Bevorzugt sind Magnetpumpen oder Pumpen mit Gleitringwellendichtung.

Als Kühler / Wärmetauscher können beispielsweise Rohr-, Platten- oder Spiralrohrwärmetauscher verwendet werden.

Durch Anhaftung des BPA-Phenol-Adduktes an der Rohrinnenseite (so genanntes Fouling) erhöht sich der Gegendruck. Dadurch verringert sich die Umwälzmenge, wodurch das Fouling noch verstärkt auftritt. Die geringere Umwälzmenge macht sich durch ein Absinken des Stromverbrauchs bemerkbar. Durch Erhöhung der Drehzahl wird die Stromaufnahme im Wärmetauscher konstant gehalten bzw. wird weniger abgesenkt. Eine Kopplung zwischen Stromaufnahme und Drehzahl ist also auch eine Kopplung zwischen Druckdifferenz und Drehzahl.

Eine erhöhte Drehzahl schon zu Beginn der Reaktion ist nicht empfehlenswert, weil eine zu hohe Geschwindigkeit dazu führt, dass die Kristalle sehr stark zerkleinert werden, wodurch die nachgeschaltete Filtration und Wäsche der BPA-Phenol-Adduktkristalle schlechter durchführbar ist, was zu Qualitätseinbußen führt. Daher wird nicht von Anfang an mit erhöhter Drehzahl der Pumpe am Wärmeaustauscher gefahren.

Nach Erhalt der BPA-Phenol-Adduktkristalle wird als letzter Schritt des erfindungsgemäßen Verfahrens das Phenol aus dem Addukt entfernt. Dies kann durch jede dem Fachmann bekannt Weise wie destillative und/oder desorptive Methoden unter Erhitzen auf Temperaturen von > 120 °C auf einen Restgehalt von < 200 ppm Phenol geschehen.

Nach der Phenolabtrennung erhält man eine Bisphenol A-Schmelze, welche ohne vorherige Verfestigung für die Herstellung von Polycarbonat nach dem Umesterungsverfahren (Schmelzepolycarbonat) verwendet werden kann. Die Bisphenol A-Schmelze kann aber auch durch bekannte Verfahren, wie z.B. nach dem Prillverfahren oder durch Abschuppung, für den Verkauf oder die Weiterverwertung verfestigt werden. Ferner kann die Schmelze in Natronlauge gelöst werden und für die Herstellung von Polycarbonat nach dem Phasengrenzflächeverfahren eingesetzt werden.

In einer anderen Verfahrensvariante wird das Phenol nur auf Restgehalte von ≤ 15% entfernt und das so erhaltene Gemisch einer weiteren Verarbeitung beispielsweise zu Polycarbonat nach dem Schmelzeverfahren zugeführt.

Die BPA-Phenol-Adduktkristalle, die aus Schritt c) erhalten werden, können alternativ auch noch einer weiteren Reinigung unterzogen werden, bevor sie in Schritt d) eingesetzt werden. Bevorzugt ist hierbei eine Reinigung durch Umkristallisation aus einer phenolhaltigen Lösung.

Nach einer Pumpendrehzahlerhöhung um 10% - 50% gegenüber dem Anfangszustand müssen die Anhaftungen im Wärmetauscher und ggf. auch im Kristaller durch bevorzugtes schnelles Erhitzen auf Temperaturen von ca. 80 °C entfernt werden, wie in DE 100 15 014 A beschrieben.

### Beispiel 1: Keine Kupplung zwischen Drehzahl (Leistungsaufnahme der Pumpe) und Druckerhöhung

Ein Produktgemisch enthaltend Bisphenol A, Nebenkomponenten und Phenol wird kontinuierlich in einen Kristaller mit Wärmetauscher, Kristallerkühler und einer Umwälzpumpe (Chemienormpumpe mit doppelter Gleitringdichtung) eingespeist und mittels der Pumpe kontinuierlich ein Teilstrom der im Kristaller vorliegenden Suspension durch den Wärmetauscher umgewälzt. Aus dem System wird kontinuierlich ein Teilstrom entnommen und der weiteren Verarbeitung zugeführt.

Folgende Charakteristika wurden beobachtet:

| | ohne Fouling (Startpunkt) | mit Fouling (Endpunkt) |
|---|---|---|
| Drehzahl der Pumpe | 550 rpm | 550 rpm |
| | | |
| Leistungsaufnahme der Pumpe | 60 kW | 40 kW |
| | | |
| Umwälzvolumen | 1500 m³/h | 500 m³/h |
| | | |
| Δ p über Wärmetauscher | 1 bar | 1,8 bar |

Der Zeitabstand zwischen zwei Maßnahmen zur Entfernung der Beläge im Kristallerkühler beträgt 22 Tage.

**Tabelle 1: Einfluss von Fouling auf das Umwälzvolumen ohne Kupplung zwischen Drehzahl der Pumpe und Druckerhöhung**

| Drehzahl der Pumpe | Δ p über Wärmetauscher | Umwälzvolumen |
|---|---|---|
| 550 | 1 | 1500 |
| 550 | 1.2 | 1280 |
| 550 | 1.3 | 1100 |
| 550 | 1.45 | 900 |
| 550 | 1.6 | 650 |
| 550 | 1.8 | 500 |

### Beispiel 2: Kupplung zwischen Drehzahl (Leistungsaufnahme der Pumpe) und Druckerhöhung

Analog Beispiel 1.

| | ohne Fouling (Startpunkt) | mit Fouling (Endpunkt) |
|---|---|---|
| Drehzahl | 550 rpm | 640 rpm |
| | | |
| Leistungsaufnahme der Pumpe | 60 kW | 74 kW |
| | | |
| Umwälzvolumen | 1500 m³/h | 1300 m³/h |
| | | |
| Δ p über Wärmetauscher | 1 bar | 1,8 bar |

Der Zeitabstand zwischen zwei Maßnahmen zur Entfernung der Beläge im Kristallerkühler beträgt 31 Tage.

**Tabelle 2: Einfluss von Fouling auf das Umwälzvolumen mit Kupplung zwischen Drehzahl der Pumpe und Druckerhöhung**

| Drehzahl der Pumpe | Δ p über Wärmetauscher | Umwälzvolumen |
|---|---|---|
| 550 | 1 | 1500 |
| 590 | 1.2 | 1420 |
| 603 | 1.4 | 1400 |
| 612 | 1.45 | 1380 |
| 630 | 1.65 | 1340 |
| 645 | 1.8 | 1300 |

## Patentansprüche

1. Verfahren zur Herstellung von Bisphenol A umfassend folgende Schritte:
a) Umsetzung von Phenol und Aceton in Gegenwart eines sulfonsauren Ionenaustauschers und eines schwefelhaltigen Cokatalysators zu einem Bisphenol A enthaltenden Produktgemisch,
b) kontinuierliche Suspensionskristallisation eines Bisphenol A-Phenol-Addukts aus dem in Schritt (a) erhaltenen Produktgemisch in einem oder mehreren Kristallern mit jeweils mindestens einem Wärmetauscher zur Kühlung und mindestens einer Pumpe, die die in den Kristallern enthaltene Suspension durch den oder die Wärmetauscher pumpt,
c) Abtrennung der so gewonnenen BPA-Phenol-Adduktkristalle durch Fest-Flüssig-Trennung,
d) Entfernung von Phenol mittels Destillation und/oder Desorption aus dem gemäß Schritt (c) erhaltenen BPA-Phenol-Addukt
**dadurch gekennzeichnet, dass** die Stromaufnahme der Pumpe(n), mit denen das Produktgemisch in Schritt b) durch die Wärmetauscher gepumpt wird, so gehalten wird, dass die Stromaufnahme um maximal ± 5% schwankt und damit mit steigendem Druckverlust über die Wärmetauscher die Drehzahl der Pumpen erhöht wird.

2. Verfahren nach Anspruch 1, wobei das Produkt aus Schritt c) mit Phenol gewaschen und/oder umkristallisiert wird.

3. Verfahren nach einem der Ansprüche 1 bis 2, bei dem das Phenol bis auf Restgehalte von < 200 ppm entfernt wird.

4. Verfahren nach einem der Ansprüche 1 bis 2, bei dem das Phenol bis auf Restgehalte von 15% entfernt wird.

5. Verfahren nach einem der Ansprüche 1 bis 4, bei dem die Drehzahl der Pumpen von Inbetriebnahme der Wärmetauscher bis zu einem Entfernen der Beläge in diesen um 10 - 50 % gegenüber der anfänglichen Drehzahl erhöht wird.

6. Verfahren nach einem der Ansprüche 1 bis 5, bei dem der umgewälzte Volumenstrom durch den / die Wärmetauscher konstant gehalten wird, so dass er um maximal ± 15% schwankt.

7. Verfahren nach einem der Ansprüche 1 bis 6, wobei die Druckdifferenz über den Wärmetauscher gemessen und mit steigender Druckdifferenz die Drehzahl der Pumpe erhöht wird.
